# EUROPEAN PATENT SPECIFICATION

(11) **EP 3 275 486 B1**
(45) Date of publication and mention of the grant of the patent: **06.09.2023**
(21) Application number: 16768851.4
(22) Date of filing: 24.03.2016
(51) Int. Cl.: A61M 5/32, A61M 5/158

(54) **MEDICAL NEEDLE, AND METHOD FOR CLOSING PROTECTOR OF MEDICAL NEEDLE**
MEDIZINISCHE NADEL UND VERFAHREN ZUM VERSCHLIESSEN DER SCHUTZEINRICHTUNG DER MEDIZINISCHEN NADEL
AIGUILLE À USAGE MÉDICAL, ET PROCÉDÉ DE BLOCAGE DE PROTECTION D'AIGUILLE À USAGE MÉDICAL

(30) Priority: 24.03.2015 JP 2015061247; 24.03.2015 JP 2015061272
(43) Date of publication of application: 31.01.2018
(73) Proprietor: Terumo Kabushiki Kaisha, Tokyo 151-0072 (JP)
(72) Inventor: YAMAZAKI, Yuusuke, Tokyo 141-0022 (JP); NAKA, Noriko, Fujinomiya-shi Shizuoka 418-0004 (JP); TABATA, Yasushi, Nagano 396-0032 (JP); AKAIKE, Nobukazu, Fujinomiya-shi Shizuoka 418-0004 (JP); TAKAGI, Yoshiki, Fujinomiya-shi Shizuoka 418-0004 (JP)
(74) Representative: Casalonga
(86) International application number: PCT/JP2016/059284
(87) International publication number: WO 2016/152948

(56) References cited:
- EP-A1- 1 254 677
- WO-A1-2014/035970
- WO-A1-2014/035970
- JP-A- H09 629
- JP-A- H1 015 074
- JP-A- S6 272 367
- JP-A- H06 261 943
- JP-A- H07 148 270
- JP-A- 2005 230 094
- JP-A- 2014 514 010
- US-A- 4 863 434
- US-A1- 2003 014 018
- US-A1- 2004 236 287

## Description

### Technical Field

The present invention relates to a medical needle including a protector that houses a needle body after puncturing a living body, and relates to a method for closing a protector of a medical needle.

### Background Art

In blood donation, puncturing a blood donor with a dedicated blood sampling needle and indwelling the needle makes a blood drawing unit. This blood drawing unit leads to a blood bag where blood is stored. The used blood sampling needle is discarded after completion of blood donation in order to prevent infection. In recent years, to prevent mispuncture of a needle tip of a blood sampling needle when discarding the same, a medical needle provided with a protector that houses a needle body has been developed and provided, for example, as disclosed in JP 3198492 B1.

In a protector of a medical needle disclosed in JP 3198492 B1, a needle body exposed at the time of puncture is retracted toward a housing space after puncture from a leading end opening of the protector so as to be stored in the housing space. In this case, the leading end opening of the protector is formed to have a relatively large diameter so that the needle body with blood attached thereto is allowed to pass through the leading end opening without touching the same.

EP1254677 discloses a passively activated safety needle assembly.

### Summary of Invention

### Technical Problem

This type of a medical needle is housed in a protector with blood attached to a needle body. Therefore, when the blood attached to the needle body is separated from the needle body and transmitted to an inner face of the protector, the blood may spatter from a leading end opening of the protector. Spattering of the blood from the medical needle in this manner contaminates medical institutions and other medical devices, which may cause a risk of infection (deterioration of hygiene) .

The present invention has been made to solve the aforementioned problem, and an object of the present invention is to provide a medical needle which considerably suppresses spattering of blood from a protector that houses a needle body so as to enhance hygiene, and to provide a method for closing a protector of a medical needle.

### Solution to Problem

The invention is as defined in claim 1. Further embodiments are defined in the dependent claims. Any aspects, embodiments, and examples of the present disclosure which do not fall under the scope of the appended claims do not form part of the invention and are merely provided for illustrative purposes.In
order to achieve the above object, a medical needle according to the present invention is provided with a needle body; and the protector configured to expose the needle body from a leading end before puncture of the needle body, including a housing space which houses the needle body after puncture by relative movement of the needle body toward a base end, wherein the housing space of the protector includes a wall portion configured to form a blood receiving portion that accumulates blood separated from the needle body by a structure of an inner face of the wall portion or by closing a leading end of the housing space with the needle body being housed in the housing space by sealing, wherein the housing space of the protector further includes a leading end portion, a diameter of a space of the leading end portion is smaller than the diameter of the housing space at the position where a needle tip is housed, and a leading end opening is formed to have a diameter so that the needle body can pass therethrough without touching the leading end opening when the needle body retracts.

According to the above description, as the wall portion included in the housing space of the protector has the blood receiving portion, even though blood attached to the needle body is separated after the needle body is housed in the housing space, the medical needle can accumulate the blood in the blood receiving portion. Therefore, with a simple configuration, the medical needle can considerably suppress the blood from spattering from the protector housing the needle body, which leads to enhancement of hygiene and reduction in inconveniences such that medical institutions and other medical devices may be contaminated with blood.

In this case, the blood receiving portion is preferably a blood reservoir provided to the entire circumference in the circumferential direction of the inner face.

### Advantageous Effects of Invention

In this manner, as the blood reservoir is provided as the blood receiving portion in the entire circumference in the circumferential direction of the inner face of the protector, no matter where the blood spatters in the inner face of the protector around the needle body when an external force such as vibration or shock is applied to the medical needle, the medical needle can receive the blood. Therefore, the medical needle can considerably suppress the spattering of the blood from the protector.

In addition to the above configuration, the blood reservoir is preferably provided to a side closer to the leading end than a needle tip of the needle body housed in the housing space.

In this manner, as the blood reservoir is provided to the side closer to the leading end than the needle tip of the needle body, it is possible to effectively accumulate the blood flowing toward the leading end of the protector.

More preferably, the blood reservoir is provided at a position in the leading end of the protector.

In this manner, as the blood reservoir is provided at the position in the leading end of the protector, even though the blood slightly spatters toward the leading end from the needle body, it is possible to accumulate the blood at the position in the leading end of the protector and to suppress the spattering of the blood more reliably.

It is preferable that the protector includes a housing cylinder having rigidity, surrounding a leading end of the needle body while the needle body is housed in the protector; and an elastic member mounted on the housing cylinder, protruding toward the leading end from the housing cylinder.

Accordingly, as the housing cylinder and the elastic member are assembled, the blood reservoir can be easily provided at the position in the leading end of the protector in the medical needle. In the medical needle, the leading end of the protector is made flexible by the elastic member so that it is possible to reduce a burden when fixing the medical needle to a living body.

Furthermore, the blood reservoir is preferably formed with a barbed portion having a ring-like shape in which a peripheral wall of the protector protrudes radially inward and toward the base end.

In this manner, as the blood reservoir is formed by the ring-like barbed portion in which the wall portion of the protector protrudes radially inward and toward the base end, it is possible to manufacture the protector (for example, shape with a mold) easily.

Still further, the blood reservoir may be provided with an absorbing portion configured to absorb the blood.

In this manner, as the absorbing portion is provided to the blood reservoir, the absorbing portion preferably absorbs the blood flowing into the blood reservoir, which suppresses the spattering of the blood from the protector more reliably.

Alternatively, the blood receiving portion is a to-be-sealed portion (sealable portion) capable of closing the leading end of the housing space by sealing.

In this manner, as the blood receiving portion is the to-be-sealed portion, the to-be-sealed portion is sealed after the needle body is housed in the housing space so that the medical needle can close the leading end of the housing space. Accordingly, it is possible to prevent more reliably the spattering of the blood held by the needle body.

In this case, the to-be-sealed portion preferably includes an elastic portion which is elastically deformable in accordance with pressing force of the sealing.

In this manner, as the to-be-sealed portion includes the elastic portion, a healthcare professional can easily press and deform the to-be-sealed portion with a general-purpose sealing device at the time of sealing, thereby closing the to-be-sealed portion.

In addition to the above configuration, it is preferable that the to-be-sealed portion is formed in a cylindrical shape, being provided with a space included as a part of the housing space in an inner portion of the to-be-sealed portion, wherein the space has a diameter smaller than a diameter of the housing space at a position where the needle tip is housed.

In this manner, the diameter of the space of the to-be-sealed portion is smaller than the diameter of the housing space at the position where the needle tip is housed. Accordingly, in the medical needle, a part of the wall portion of the to-be-sealed portion is easily brought into contact with another part thereof at the time of sealing, which leads to a preferable sealing.

It is preferable that the protector includes a housing cylinder having rigidity, surrounding a leading end of the needle body while the needle body is housed in the protector; and an elastic member mounted on the housing cylinder, including the to-be-sealed portion protruding toward the leading end from the housing cylinder.

In this manner, as the protector includes the housing cylinder and the elastic member, exposure of the needle body can be prevented by the rigid housing cylinder, and the elastic member or the to-be-sealed portion can be easily sealed by the healthcare professional, which causes a closed state of the housing space.

Furthermore, in order to achieve the above object, the present invention provides a method for closing a protector of a medical needle provided with a needle body; and the protector configured to expose the needle body from a leading end before puncture of the needle body, including a housing space which houses the needle body after puncture by relative movement of the needle body toward a base end, the method including: sealing a to-be-sealed portion including a wall portion of the protector provided to a side closer to the leading end than a needle tip of the needle body with the needle body being housed inside the housing space so as to close a leading end of the housing space.

According to a medical needle and a method for closing a protector of a medical needle according to an embodiment of the present invention, it is possible to considerably suppress spattering of blood from the protector housing a needle body with a simple configuration, which leads to further enhancement of hygiene.

### Brief Description of Drawings

Fig. 1 is a perspective view illustrating a medical needle in use according to a first embodiment of the present invention.
Fig. 2 is an exploded perspective view illustrating the medical needle of Fig. 1.
Fig. 3A is a perspective view illustrating the medical needle before use; Fig. 3B is a perspective view illustrating the medical needle in use; and Fig. 3C is a perspective view illustrating the medical needle after use.
Fig. 4A is a cross-sectional view with a partial plan view of the medical needle in use; and Fig. 4B is a cross-sectional view taken along the line IVB-IVB of Fig. 4A.
Fig. 5A is a plan view of the medical needle after use; and Fig. 5B is a cross-sectional view taken along the line VB-VB of Fig. 5A.
Fig. 6A is a first cross-sectional view for describing spattering of blood in the medical needle after use; and Fig. 6B is a second cross-sectional view for describing a flow of the blood following Fig. 6A.
Fig. 7A is a cross-sectional view illustrating a protector according to a first modification; and Fig. 7B is a cross-sectional view illustrating a protector according to a second modification.
Fig. 8 is a perspective view illustrating a medical needle in use according to a second embodiment of the present invention.
Fig. 9A is a perspective view illustrating the medical needle of Fig. 8 after use in which a needle body is housed in a protector; and Fig. 9B is a perspective view illustrating the medical needle of Fig. 8 in which a leading end portion of the protector is sealed.
Fig. 10 is an enlarged perspective view illustrating a clamp that closes a leading end portion of the medical needle.
Fig. 11A is a side cross-sectional view enlarging a leading end of the medical needle at the time of sealing; and Fig. 11B is a side cross-sectional view enlarging the leading end of the medical needle after sealing.

### Description of Embodiments

Hereinafter, a preferred embodiment of a medical needle and a method for closing a protector of a medical needle according to the present invention will be described in detail with reference to the accompanying drawings.

### [First Embodiment]

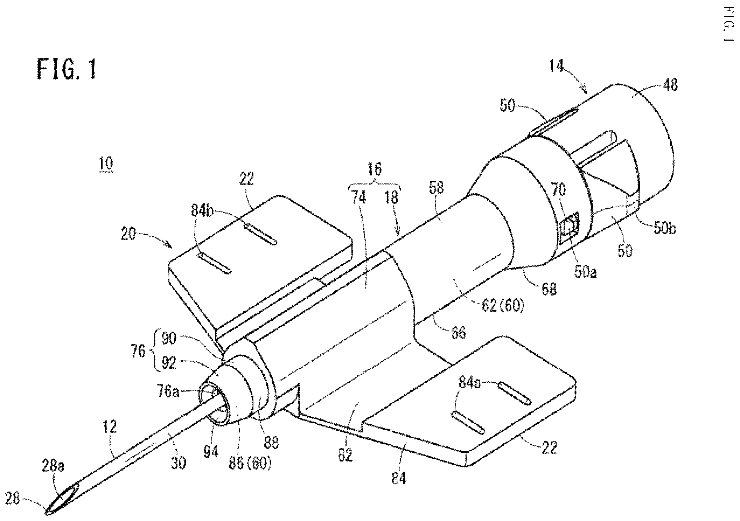

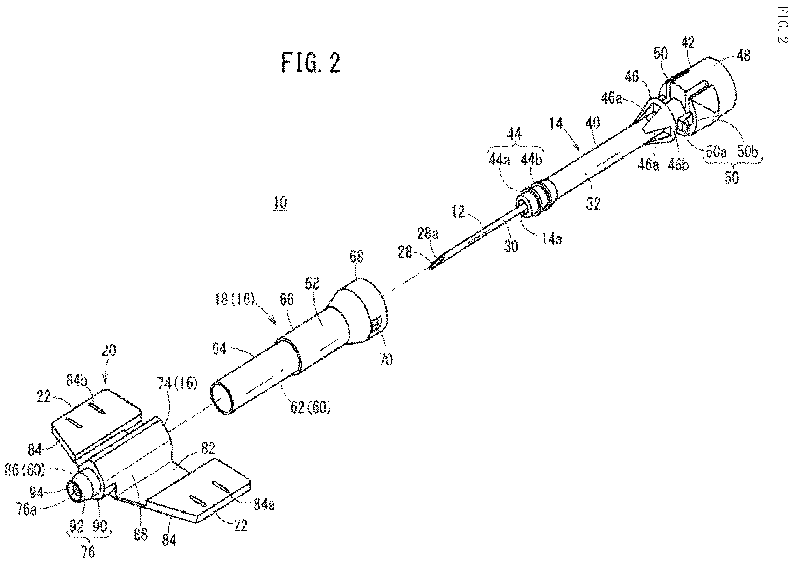

A medical needle 10 according to a first embodiment of the present invention is a blood sampling needle for whole blood which is used for blood donation and for collecting a specific amount of all the blood components of a blood donor. As illustrated in Fig. 1 and Fig. 2, the medical needle 10 includes a needle body 12 (cannula) ; a hub 14 (needle holding portion) configured to hold the needle body 12; and a protector 16 configured to house the needle body 12 after puncturing the blood donor. A configuration of the medical needle 10 according to this embodiment is not limited to a blood sampling needle for whole blood. The medical needle 10 may be applied, for example, to ablood sampling needle for apheresis or a needle for injecting a pharmaceutical liquid.

Furthermore, the medical needle 10 is a winged indwelling needle (what is called a butterfly needle). A pair of wings 22 of the medical needle 10 serves as a gripping portion which a healthcare professional such as a doctor and a nurse grips so as to perform puncture of the needle body 12. The pair of wings 22 also serves as a fixing portion which is stretched out on a body surface of the blood donor and fixed with a tape or a bandage during blood donation. The protector 16 of the medical needle 10 includes a housing cylinder 18; and a shaft 74 of a wing member 20 (elastic member) attached to the housing cylinder 18. The wings 22 are formed on the shaft 74 in an integrated manner. Note that it is possible to apply the medical needle 10 according to this embodiment as a needle other than a winged needle.

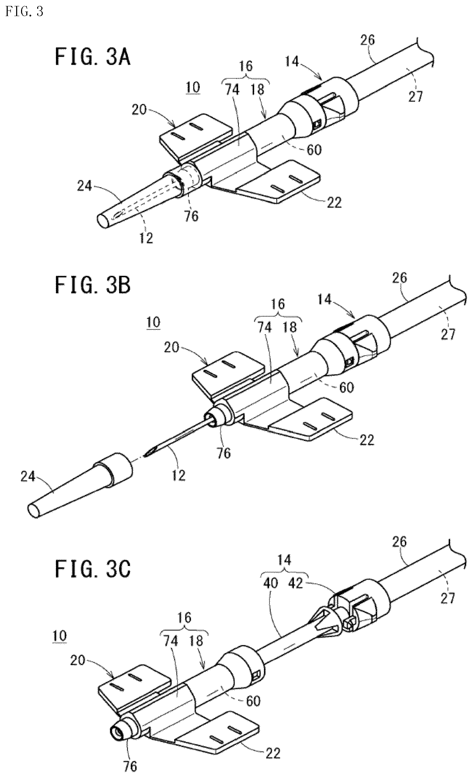

The medical needle 10 is a part of a blood sampling set in a state of product offering (hereinafter also referred to as "before use") illustrated in Fig. 3A. Specifically, while the hub 14 and the protector 16 are being attached to each other, a tube 26 connected to a blood bag (not illustrated) is connected and fixed to a base end of the hub 14. In the medical needle 10, the needle body 12 protrudes from a leading end of the protector 16, and the needle body 12 is covered with a cap 24.

In blood sampling (hereinafter also referred to as "in use"), the healthcare professional removes the cap 24 as illustrated in Fig. 3B, and punctures a blood vessel of the blood donor with the exposed needle body 12. Accordingly, the medical needle 10 serves as a blood drawing unit for collecting blood of the blood donor, causing the blood to flow into an inner cavity 27 of the tube 26, thereby supplying the blood to the blood bag through the inner cavity 27.

After completion of blood sampling (hereinafter also referred to as "after use"), the healthcare professional withdraws the needle body 12 from the blood donor. Then, the needle body 12 is housed in the protector 16 as illustrated in Fig. 3C, and discarded. Hereinafter, a configuration of each part in the medical needle 10 that performs the above operation will be described in detail.

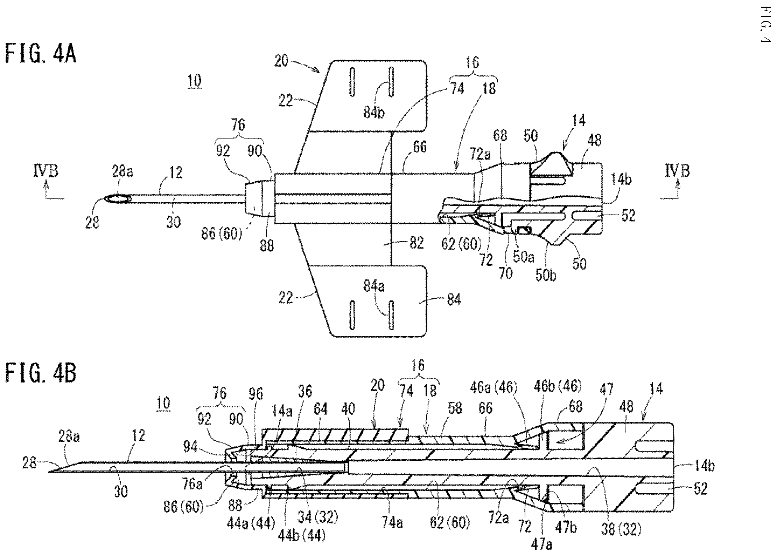

As illustrated in Figs. 1, 4A, and 4B, the needle body 12 of the medical needle 10 is an elongated hollow tube having rigidity that can be inserted into the blood vessel from the skin of the blood donor. The needle body 12 is formed with a sharp needle tip 28 at a leading end thereof, and the needle tip 28 is provided with an opening 28a through which the blood flows. The needle body 12 is also provided with a flow passage 30 in an inner portion thereof. The flow passage 30 is communicated with the opening 28a, allowing the blood to flow along an axial direction of the needle body 12.

Although a thickness of the needle body 12 is not particularly limited, it is preferable to design the needle body 12 in consideration of time shortening of blood donation and reduction in fear of a blood donor. In this embodiment, the needle body 12 is set to have an outer diameter of 1.25 mm. Furthermore, a length of the needle body 12 is preferably designed in consideration of improvement in operability and reduction in fear of a patient. For example, it is preferable that a length of a portion protruding from the hub 14 is 40 mm or less. An example of a constituent material of the needle body 12 includes a metallic material such as stainless steel, aluminum or aluminum alloy, and titanium or titanium alloy.

As illustrated in Figs. 2, 4A, and 4B, the hub 14 holding the needle body 12 is formed in a cylindrical shape, having a shaft center coaxial with a shaft center of the needle body 12, and the hub 14 is where a base end portion of the needle body 12 is connected and fixed. Before use and in use, this hub 14 is engaged with the protector 16 and can be handled in an integrated manner with the protector 16. After use, the engagement between the hub 14 and the protector 16 is released so that the hub 14 is retracted relative to the protector 16, which causes the needle body 12 to be disposed inside the protector 16.

An inner portion of the hub 14 is provided with a hollow portion 32 passing through the shaft center, leading to opening portions 14a, 14b in both ends of the hub 14. In a side close to a leading end of the hollow portion 32, an inner wall of the hub 14 forms a tapered portion 34 whose diameter becomes smaller toward a base end from the opening portion 14a disposed in a front end. An adhesive 36 is injected into an inner portion of the tapered portion 34 so that the needle body 12 is firmly fixed. The hollow portion 32 in a side closer to the base end than the tapered portion 34 is formed to have a slightly large diameter, serving as a communicating passage 38 communicated with the flow passage 30 of the fixed needle body 12.

The hub 14 is formed by integrally molding a shaft portion 40 housed inside the protector 16 before use and in use and an operating portion 42 connected to a base end of the shaft portion 40, being exposed to the outside of the protector 16. The shaft portion 40 is slidably disposed inside the protector 16, having a length slightly longer than an axial length of the protector 16. On an outer periphery of the shaft portion 40, a locking portion 44 is provided at a position close to a leading end, and a closing portion 46 is provided at a position close to a base end.

The locking portion 44 includes an annular projection 44a and an annular hook 44b. The annular projection 44a protrudes radially outward, being disposed in a side close to the leading end. The annular hook 44b protrudes radially outward, leaving a space toward the base end from the annular projection 44a. The annular projection 44a is formed in a quadrangular shape in a cross-sectional view (see Fig. 4B) . The annular hook 44b is formed in a substantially right triangular shape in a cross-sectional view, having a slope inclined toward the base end.

The closing portion 46 is formed in a conical shape on the outer periphery of the shaft portion 40, having a diameter that gradually increases toward the base end of the hub 14. The closing portion 46 has a plurality of (four) notched portions 46a along a circumferential direction of the shaft portion 40. The notched portions 46a extend from a leading end of the conical shaped closing portion 46 to a base end thereof so as to expose the outer periphery of the shaftportion40. Apart of the closing portion 46 closer to the base end than the notched portions 46a is a closing wall 46b having a disk-like shape, being disposed continuously through the entire circumference in the circumferential direction of the shaft portion 40. The closing wall 46b comes into contact with a peripheral wall 58 (wall portion) included in a housing space 60 when the hub 14 is housed inside the protector 16.

The operating portion 42 includes a base portion 48 and a pair of arms 50. The base portion 48 is connected to the shaft portion 40, having a narrow leading end and a wide base end in a plan view (see Fig. 4A) . The pair of arms 50 protrudes from the base end of the base portion 48 toward the leading end. A base end surface of the base portion 48 is provided with the opening portion 14b and a connecting groove 52. The opening portion 14b is disposed in the base end communicated with the hollow portion 32 at a position passing through the shaft center. The connecting groove 52 has an annular shape, circling around an outer periphery of the opening portion 14b in a radial direction. The connecting groove 52 is engaged with the tube 26 (see Fig. 3A) so as to hold the same and to communicate the communicating passage 38 of the hub 14 and the inner cavity 27 of the tube 26. Note that the base portion 48 may be formed integrally with the hub 14 as illustrated in the drawing, or may be formed separately.

Each arm 50 includes a claw portion 50a and an operating projection 50b. The claw portion 50a protrudes outward in a width direction, being disposed in a side close to a leading end. The operating projection 50b protrudes outward in the width direction, being disposed in a side close to a base end with a space being left from the claw portion 50a. Before use and in use, the claw portions 50a are inserted into the protector 16 so as to be engaged with engagement holes 70 of the protector 16. Accordingly, the hub 14 and the protector 16 are combined.

The operating projections 50b are parts exposed from the protector 16 and pressed by the healthcare professional. In other words, the pair of arms 50 elastically deforms inward as the healthcare professional presses a pair of operating projections 50b. Accordingly, the claw portions 50a in the leading end come close to each other to release the engagement between the hub 14 and the protector 16, which causes the hub 14 to retract with respect to the protector 16 together with the needle body 12.

It is preferable that the hub 14 is made of a relatively hard resin material, for example, a thermoplastic resin such as polypropylene, polycarbonate, polyamide, polysulfone, and polyarylate as well as polyvinyl chloride. As a material of the hub 14 in this embodiment, polycarbonate is employed which is a material having high heat resistance, hygiene, and water repellency.

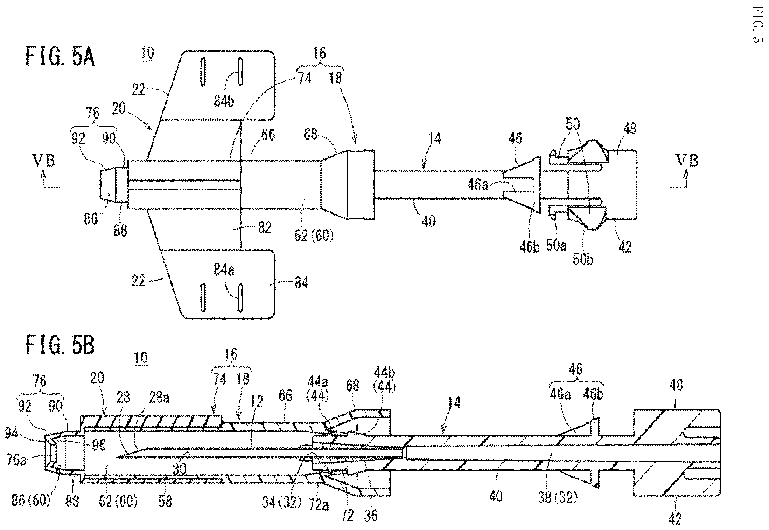

As illustrated in Figs. 2 and 3C, the protector 16 of the medical needle 10 has a function of housing the needle body 12 after completion of blood donation (after use) so as to prevent mispuncture of the needle tip 28. The housing cylinder 18 of the protector 16 is formed by the peripheral wall 58 having a cylindrical shape, and an inner portion of the housing cylinder 18 includes a first space 62 which is a part of the housing space 60 for the needle body 12. An axial length of the housing cylinder 18 is formed to be longer than an axial length of a portion of the needle body 12 protruding from the hub 14 so that the needle tip 28 is positioned within the first space 62 when the needle body 12 is housed (see also Fig. 5B) . As a constituent material of the housing cylinder 18, it is preferable to appropriately employ the resin materials mentioned above as the material of the hub 14. Similar to the hub 14, polycarbonate is employed as a constituent material of the housing cylinder 18 in this embodiment.

As illustrated in Fig. 2, the housing cylinder 18 includes a mounting cylindrical portion 64, an extending cylindrical portion 66, and a locking cylindrical portion 68. The mounting cylindrical portion 64 is used for mounting the wing member 20. The extending cylindrical portion 66 leads to a base end of the mounting cylindrical portion 64, extending toward a base end. The locking cylindrical portion 68 leads to a base end of the extending cylindrical portion 66, being disposed outside of the housing cylinder 18 in a radial direction with a slightly large diameter.

The mounting cylindrical portion 64 is formed to be slightly thinner than the extending cylindrical portion 66, and the wing member 20 is tightly adhered and fixed to an outer periphery of the mounting cylindrical portion 64. A step between the mounting cylindrical portion 64 and the extending cylindrical portion 66 defines a position where the wing member 20 is to be mounted. While the hub 14 is housed in the first space 62, the annular projection 44a and the annular hook 44b are positioned close to the peripheral wall 58 in a leading end of the mounting cylindrical portion 64 so that the shaft center of the hub 14 is reliably coaxial with the shaft center of the housing cylinder 18, which stabilizes the posture of the needle body 12.

The locking cylindrical portion 68 is formed to have a diameter reversely tapered toward its base end from a boundary of the extending cylindrical portion 66, and an outer diameter unvaried from a midway position to the base end. A pair of engagement holes 70 is formed in the peripheral wall 58 of the locking cylindrical portion 68 in order to hook the claw portions 50a of the hub 14. Note that a locking structure of the protector 16 and the hub 14 is not particularly limited. For example, the peripheral wall 58 may be provided with recesses or grooves capable of hooking the claw portions 50a.

As illustrated in Fig. 4B, a tapered inner face 47b of the locking cylindrical portion 68 is brought into contact with a surface of an inclined protruding end face 47a of the closing wall 46b of the hub 14 through the entire circumference in the circumferential direction. In other words, the protruding end face 47a of the hub 14 and the inner face 47b of the housing cylinder 18 are included in a closing structure 47 that closes the housing space 60 in a side closer to the leading end than the closing wall 46b.

The housing cylinder 18 also includes a plurality (four in this embodiment) of elastic pieces 72 disposed in an inner boundary of the extending cylindrical portion 66 and the locking cylindrical portion 68. The elastic pieces 72 protrude toward a base end of the first space 62 and toward a shaft center thereof. The four elastic pieces 72 are provided at equal intervals (out of phase with each other by 90 degrees) along a circumferential direction of the protector 16. Each elastic piece 72 is shaped so that a protruding end thereof elastically comes close to the shaft center of the housing cylinder 18. Furthermore, a predetermined position on a surface of each elastic piece 72 facing the shaft center (a position close to a part connecting with the peripheral wall 58) is formed with an engagement groove 72a that is engaged with the annular projection 44a when the hub 14 retracts.

Before use and in use, the four elastic pieces 72 are housed in the four notched portions 46a formed in the closing portion 46 of the hub 14. Accordingly, the hub 14 is restricted to rotate relative to the protector 16 and is prevented from rattling due to the elastic pieces 72 on four sides.

Returning to Fig. 1, the wing member 20 of the protector 16 is firmly fixed to the mounting cylindrical portion 64 of the housing cylinder 18. The wing member 20 is made of a resin material having higher flexibility and lower modulus of elasticity than those of the housing cylinder 18 (that is, the wing member 20 includes an elastic body). Examples of the elastic body included in the wing member 20 include various rubber materials (particularly, vulcanized rubber materials) such as natural rubber, isoprene rubber, butyl rubber, chloroprene rubber, nitrile-butadiene rubber, styrene-butadiene rubber, and silicone rubber; styrene elastomers; hydrogenated styrene type elastomers; these styrene elastomers mixed with polyolefins such as polyethylene, polypropylene, polybutene, and α-olefin copolymer; the aforementioned resins mixed with oil such as liquid paraffin, and process oil or mixed with powder inorganic materials such as talc, cast, and mica. Example of the elastic body further include polyvinyl chloride elastomers; olefin elastomers; polyester elastomers; polyamide elastomers; polyurethane elastomers; and mixtures thereof. The wing member 20 is formed more flexibly than the housing cylinder 18 so that it is possible to weaken pressure on the body surface of the blood donor while indwelling the medical needle 10, which reduces burden on the blood donor.

As illustrated in Figs. 1 to 4B, the wing member 20 includes the shaft 74, the pair of wings 22, and a leading end portion 76. The shaft 74 is configured to be mounted on the mounting cylindrical portion 64. The pair of wings 22 leads to a lower portion of the shaft 74 (a part to be fixed on the body surface), protruding outward in a width direction. The leading end portion 76 protrudes toward the leading end from the shaft 74.

As described above, the shaft 74 of the wing member 20 is a part of the protector 16. The shaft 74 is formed to be thicker than the mounting cylindrical portion 64 of the housing cylinder 18 and to have a length equivalent to an axial length of the mounting cylindrical portion 64. An inner portion of the shaft 74 is provided with a mounting hole 74a which is tightly adhered to the outer periphery of the mounting cylindrical portion 64.

The pair of wings 22 has thin-wall portions 82 and outer wing portions 84. The thin-wall portions 82 are connected to the shaft 74 and formed to be thin. The outer wing portions 84 lead to projecting sides of the thin-wall portions 82 and formed to be thicker than the thin-wall portions 82. Under a normal condition where the wings 22 are not operated by the healthcare professional, the pair of wings 22 protrude outward in the width direction of the shaft 74. At the time of puncture of the needle body 12, the healthcare professional puts a pair of outer wing portions 84 together and pinches the same above the shaft 74 so as to preferably perform puncture of the needle body 12.

Therefore, the thin-wall portions 82 are configured to be elastically deformable with a weak force, and upper surfaces of the thin-wall portions 82 deform toward the shaft 74 at the time of puncture . Edges in leading ends of the thin-wall portions 82 and the outer wing portions 84 are inclined toward base ends thereof so that the needle body 12 and the leading end portion 76 become readily visible at the time of puncture.

Furthermore, the pair of outer wing portions 84 is formed in a rectangular shape in a plan view, protruding toward the base end further than the thin-wall portions 82. One of opposing surfaces at which the pair of outer wing portions 84 come into contact with each other is formed with a plurality (two in this embodiment) of protrusive portions 84a elongated in a width direction of the wing member 20. The other side of the opposing surfaces is formed with a plurality (two) of recessed portions 84b elongated in the width direction and formed at positions corresponding to the protrusive portions 84a. Therefore, in the wing member 20, it is possible to prevent displacement of the outer wing portions 84 when the pair of outer wing portions 84 are superposed.

The leading end portion 76 is a cylindrical portion protruding toward the leading end from a leading end surface of the shaft 74. This leading end portion 76 is mounted with the cap 24 illustrated in Fig. 3A before use. An inner portion of the leading end portion 76 is formed with a second space 86 which is communicated with the first space 62 and included in the housing space 60 together with the first space 62 in a state where the wing member 20 is mounted on the housing cylinder 18.

The leading end portion 76 has a peripheral wall 88 (wall portion) that surrounds the second space 86 in a circumferential direction. The peripheral wall 88 includes a connecting portion-in-base end 90 that leads to the shaft 74; and a tapered portion-in-leading end 92 that is formed to have a slightly tapered shape at a leading end of the connecting portion-in-base end 90. Before use and in use, a part of the hub 14 closer to its leading end than the annular projection 44a is inserted into the second space 86 in the connecting portion-in-base end 90. In other words, the peripheral wall 88 of the connecting portion-in-base end 90 is supported by the leading end of the hub 14 from the inside of the peripheral wall 88. In this state, the cap 24 is attached to the outside of the leading end portion 76 before use.

A leading end of the tapered portion-in-leading end 92 is provided with a barbed portion 94 which is inclined inward from the peripheral wall 88 and is formed in a bowl-like shape. A protruding end of this barbed portion 94 forms a leading end opening 76a that is communicated with the second space 86. The leading end opening 76a is formed to have a relatively large diameter so that the needle body 12 can pass therethrough without touching the leading end opening 76a when the needle body 12 retracts. A diameter of the leading end opening 76a depends on the outer diameter of the needle body 12. When the diameter of the leading end opening 76a is set to form a gap, for example, of 0.3 mm or more between the needle body 12, the needle body 12 can preferably pass through the leading end opening 76a without touching the same. In this embodiment, the diameter of the leading end opening 76a is set to 2 mm.

The barbed portion 94 is formed to have an annular shape along the entire circumference in the circumferential direction of the leading end portion 76. The barbed portion 94 diagonally protrudes inward in a radial direction and toward a base end so as to form an annular recess 96 in a leading end of the second space 86 along the entire circumference in the circumferential direction. Furthermore, the barbed portion 94 reinforces a part closest to the leading end of the protector 16 so as to prevent the wing member 20 from being torn at its leading end. An end edge of the barbed portion 94 included in the leading end opening 76a is formed in parallel with the axial direction of the needle body 12.

When blood flows from the first space 62 in a side close to the base end or from the second space 86 toward the leading end, the annular recess 96 functions as a blood reservoir (blood receiving portion) that accumulates the blood. Accordingly, the medical needle 10 suppresses spattering of the blood from the leading end opening 76a even when the blood attached to the needle body 12 is separated therefrom inside the housing space 60. Since the annular recess 96 is positioned at a part closest to the leading end of the protector 16, circulating around the entire circumference in the circumferential direction of the second space 86, no matter how the blood spatters around the housing space 60, the annular recess 96 can easily receive the blood.

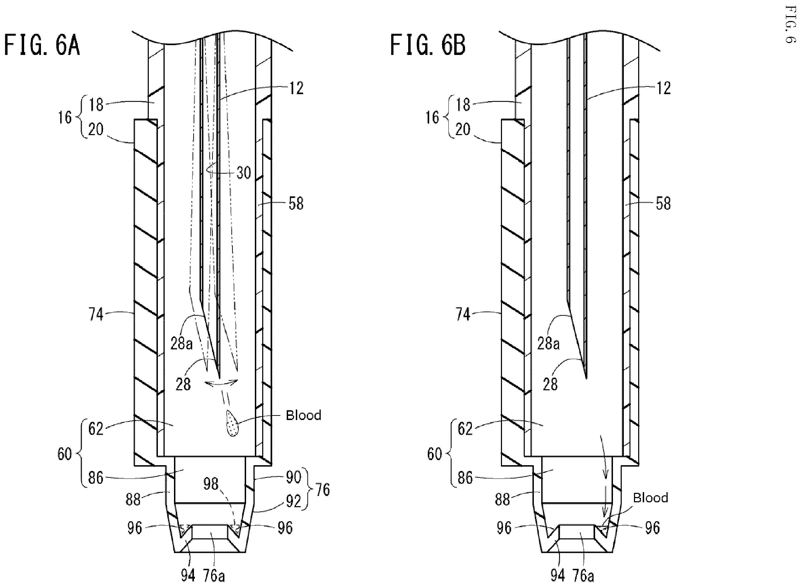

Furthermore, since the annular recess 96 is formed to have a bottom portion with a corner smaller than 90 degrees in a cross-sectional side view, it is possible to preferably suppress the spattering of the accumulated blood. An amount of protrusion of the barbed portion 94 from the peripheral wall 88 (a capacity of the annular recess 96) is not particularly limited, but may be large enough to accumulate a small amount of blood. An absorbing portion 98 (see Fig. 6A) capable of absorbing blood may be provided inside the annular recess 96. The absorbing portion 98 can be made of, for example, a porous body, and a fiber material. Accordingly, it is possible to favorably absorb the blood flowing into the annular recess 96, and to suppress more reliably the spattering of the blood from the leading end opening 76a.

The medical needle 10 according to this embodiment is basically configured as described above, and its function effect will now be described.

The medical needle 10 is provided as a blood sampling set used for blood donation of whole blood. Before use, the arms 50 of the hub 14 and the engagement holes 70 of the housing cylinder 18 are engaged with each other, and the wing member 20 is fixed to the mounting cylindrical portion 64 of the housing cylinder 18 so that the hub 14 and the protector 16 can be handled in an integrated manner. In this state of assembly, the needle body 12 protrudes from the leading end portion 76 of the wing member 20. In the state of product offering, the needle body 12 is covered by the cap 24 attached to the leading end portion 76.

In use, a healthcare professional removes the cap 24 and punctures the blood vessel of the blood donor with the needle body 12. The needle body 12 inserted into the blood vessel causes the blood to flow out through the opening 28a and the flow passage 30. This blood is stored in the blood bag through the communicating passage 38 of the hub 14 and the inner cavity 27 of the tube 26. During blood donation, the wing member 20 is applied and fixed to a body surface of a blood donor with a tape and the like.

After completion of blood donation (after use), the healthcare professional removes the needle body 12 from the body surface of the blood donor. The healthcare professional then presses the operating projections 50b of the pair of arms 50 to release the engagement between the protector 16 and the hub 14, and retracts the hub 14 relative to the protector 16 so as to guide the needle body 12 to the housing space 60 of the protector 16. At this time, since the leading end opening 76a of the wing member 20 is formed to be relatively large, a puncturing part of the needle body 12 can pass through the leading end opening 76a without touching the barbed portion 94, which reduces necessities to wipe off the blood attached around the needle body 12. Thus, it is possible to maintain hygiene of an outer surface of the wing member 20.

As illustrated in Figs. 5A and 5B, when the locking portion 44 of the hub 14 reaches the elastic pieces 72 of the protector 16 at the time of retraction of the hub 14, the annular projection 44a is inserted into the engagement grooves 72a of the elastic pieces 72, which restricts retraction of the hub 14. Accordingly, the needle body 12 is housed inside the protector 16. With the needle body 12 housed in the housing space 60, the medical needle 10 covers the needle tip 28 of the needle body 12 with the rigid housing cylinder 18. The medical needle 10 also restricts re-movement of the hub 14 toward the leading end as the annular hook 44b of the hub 14 hooks with the elastic pieces 72 of the housing cylinder 18. Therefore, it is possible to prevent mispuncture due to the needle tip 28 being re-exposed from the protector 16.

Herein, the medical needle 10 is discarded with the needle body 12 being housed in the protector 16. Particularly, when the medical needle 10 is vibrated or shocked at the time of storage or the like before disposal, the needle body 12 rocks as illustrated in Fig. 6A. In a case, for example, where the medical needle 10 is held with the protector 16 and the needle body 12 facing downward, the needle tip 28 of the needle body 12 rocks widely, having a part where the protector 16 and the hub 14 are locked as a base point. Accordingly, the blood attached to the needle body 12 is likely to spatter around. Alternatively, in a case where the medical needle 10 stands obliquely at the time of disposal, the blood drips from the needle tip 28 to the peripheral walls 58, 88.

The blood separated from the needle body 12 attaches to the peripheral walls 58, 88 around the housing space 60 and flows to the leading end of the protector 16 through along the peripheral walls 58, 88. Since the housing cylinder 18 is made of polycarbonate, when the blood attaches to the housing cylinder 18, the blood is easily moved toward the leading end due to its water repellency.

Herein, the medical needle 10 according to this embodiment forms the annular recess 96 by the barbed portion 94 provided in the entire circumference in the circumferential direction of an inner face in the leading end of the protector 16. Accordingly, as illustrated in Fig. 6B, the blood flowing toward the leading end is accumulated in the annular recess 96. Furthermore, the annular recess 96 blocks the blood from advancing radially inward due to the barbed portion 94 protruding radially inward and toward the base end, and the annular recess 96 allows the blood to flow along a circumferential direction of the annular recess 96. Thus, it is possible to preferably prevent spattering of the blood from the leading end opening 76a of the protector 16.

As described above, the medical needle 10 according to the first embodiment has the annular recess 96 that accumulates the blood around the entire circumference in the circumferential direction of the inner face of the protector 16. Therefore, even when the blood attached to the needle body 12 spatters, it is possible to accumulate the blood in the annular recess 96. In particular, the blood attached to the needle body 12 is likely to spatter toward the peripheral walls 58, 88 of the protector 16 when an external force such as vibration or shock is applied to the medical needle 10, but it is difficult to predict an orientation of the spattering blood. On the other hand, the medical needle 10 has the annular recess 96 on the entire circumference in the circumferential direction of the peripheral wall 88 (inner face) of the leading end portion 76 so that it is possible to receive the spattering blood no matter where the blood may spatter around the needle body 12. Therefore, the medical needle 10 can considerably suppress the spattering of the blood from the protector 16, which leads to enhancement of hygiene and reduction in inconveniences such that medical institutions and other medical devices may be contaminated with blood.

In this case, the annular recess 96 is provided in a side closer to the leading end than the needle tip 28 of the needle body 12 so that it is possible to efficiently accumulate the blood flowing toward the leading end of the protector 16. Furthermore, since the annular recess 96 is provided at a part closest to the leading end of the protector 16, even when the blood slightly spatters toward the leading end from the needle body 12, it is possible to accumulate the blood at a position in the leading end of the protector 16, which suppresses the spattering of the blood more reliably. Still further, since the annular recess 96 is formed by the ring-shaped barbed portion 94 in which the peripheral wall 88 of the wing member 20 protrudes radially inward and toward the base end, it is possible to manufacture the protector 16 (for example, shape with a mold) easily. Assembling the wing member 20 and the housing cylinder 18, the annular recess 96 can be easily provided at the position in the leading end of the protector 16 in the medical needle 10.

The medical needle 10 according to this embodiment is not limited to the above embodiment, and various modifications and applications may be employed. In the following description, it should be noted that the same reference numerals are given to components having the same configuration or the same functions as those of the medical needle 10 according to this embodiment, and a detailed description thereof will be omitted.

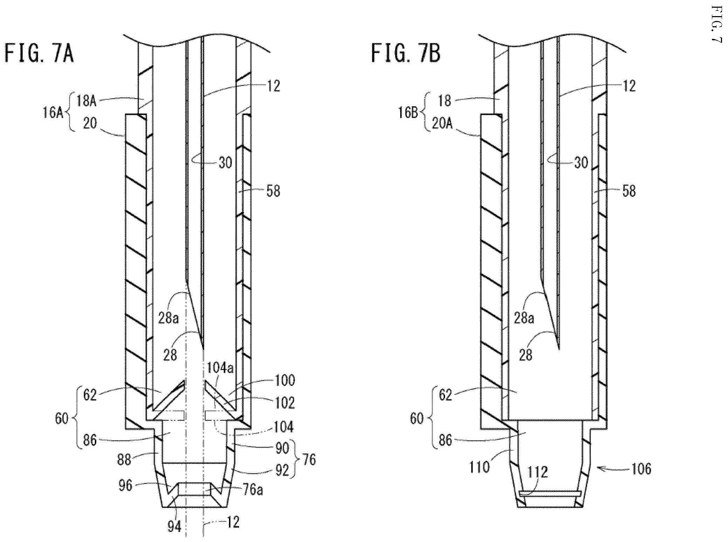

A protector 16A according to a first modification illustrated in Fig. 7A differs from the protector 16 according to this embodiment in that an annular recess 100 configured to accumulate blood spattering from the needle body 12 is provided to a housing cylinder 18A of the protector 16A. In this case, if the housing cylinder 18A is provided with a barbed portion 102 that allows the needle body 12 to pass therethrough, the needle body 12 may come into contact with the barbed portion 102 at the time of retraction of the needle body 12, and blood may be attached to the barbed portion 102. This is because the wing member 20 covers around of the barbed portion 102 so that it is possible to conceal the attachment of the blood to the barbed portion 102. Accordingly, the barbed portion 102 can protrude to a vicinity of an outer periphery of the needle body 12, which preferably suppresses spattering of the blood. Similar to this embodiment, Fig. 7A illustrates the wing member 20 including the barbed portion 94, but the wing member 20 may not include the barbed portion 94. Furthermore, the blood reservoir is not limited to the barbed portion 94 having a V-shaped cross-section, but may be formed with a flat space 104a by a flat portion 104 having an L-shaped cross section orthogonal to an axial direction of the housing cylinder 18A as indicated by two-dot chain lines in Fig. 7A.

A protector 16B according to a second modification illustrated in Fig. 7B differs from the protector 16 according to this embodiment in that a blood reservoir is formed by an annular groove 112 having a narrow width, being formed along a circumferential direction of a peripheral wall 110 of a projecting portion 106 (a wing member 20A) . In such a manner, as the annular groove 112 is disposed in midstream of a flow of blood in the peripheral wall 110, the blood flows into the annular groove 112. In particular, as the annular groove 112 is narrow, it is also possible to accumulate the blood preferably by a capillary action. It should be noted that a plurality of annular grooves 112 may be formed along an axial direction of the protector 16B. Furthermore, the annular groove 112 may be provided in the housing cylinder 18.

### [Second Embodiment]

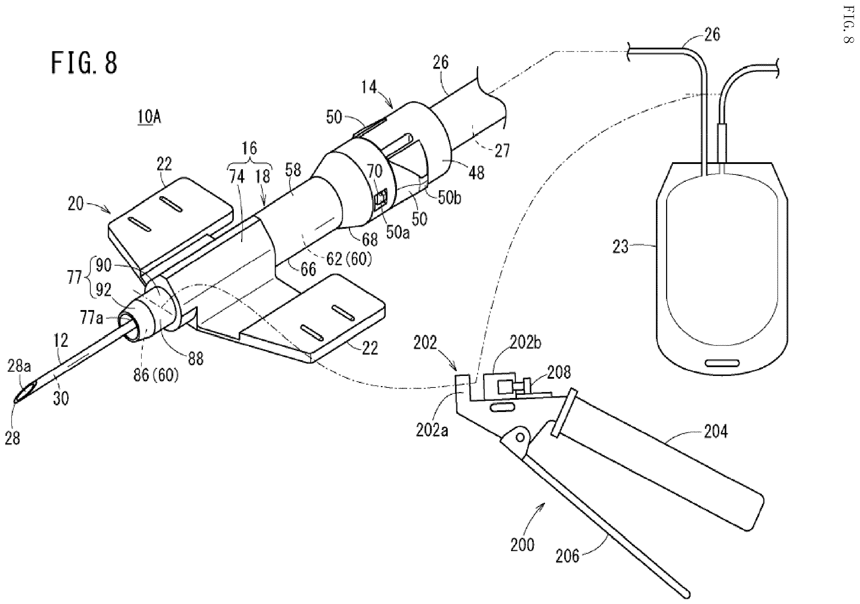

Next, a medical needle 10A according to a second embodiment of the present invention will be described with reference to Figs. 8 to 11B. This medical needle 10A is similar to the medical needle 10 in that it is configured as an indwelling needle including a needle body 12, a hub 14, and a protector 16, but is different from the medical needle 10 in that a leading end portion 77 is closed when the needle body 12 is housed in the protector 16.

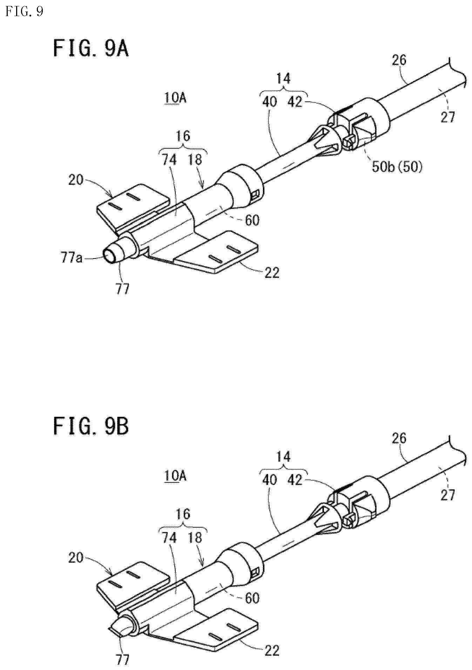

In this case, the medical needle 10A is used by a handling procedure similar to the medical needle 10 before use and in use. After use, as illustrated in Fig. 9A, a healthcare professional operates a sealing device 200 (see Fig. 8) to cut and weld a tube 26 close to a blood bag 23, and the needle body 12 is withdrawn from a blood donor so that the needle body 12 is housed inside the protector 16. Furthermore, in the medical needle 10A, as illustrated in Fig. 9B, the leading end portion 77 of the protector 16 is sealed by the sealing device 200 (see Fig. 8) which the healthcare professional holds.

Note that the meaning of the term "seal" in this specification includes various concepts for closing a spatial inner face in an airtight manner or liquid tight manner, for example, not only to cut and weld the leading end portion 77 simultaneously but also to simply weld, to bond, or to close the leading end portion 77 by pressurizing (including a case of closing the leading end portion 77 by sandwiching). Furthermore, the term "sealing" in this specification indicates that the leading end portion 77 (to-be-sealed portion) is closed with an artificial seal at least in a state of liquid-tightness. The medical needle 10A is discarded after sealing the protector 16.

Specifically, the leading end portion 77 of the protector 16 is formed in a cylindrical shape, protruding from the leading end surface of a shaft 74 toward a leading end. Before use, the leading end portion 77 is mounted with the cap 24 described above (see Fig. 3A) . The leading end portion 77 has a peripheral wall 88 (wall portion) that surrounds a second space 86 in a circumferential direction. This peripheral wall 88 has a connecting portion-in-base end 90 that leads to the shaft 74; and a tapered portion-in-leading end 92 that has a slightly tapered shape, being formed at a leading end of the connecting portion-in-base end 90. Before use and in use, a part of the hub 14 closer to its leading end than the annular projection 44a is inserted into the second space 86 in the connecting portion-in-base end 90.

The leading end portion 77 of a wing member 20 is included in a to-be-sealed portion which is to be sealed by the sealing device 200 when the needle body 12 is housed in a first space 62 of a housing cylinder 18 after use. Therefore, a diameter of the second space 86 (connecting portion-in-base end 90) of the leading end portion 77 is set to be smaller than a diameter of the first space 62. More preferably, the diameter of the second space 86 is substantially equivalent to a diameter of an inner cavity 27 of the tube 26 or less than the diameter of the inner cavity 27. As the diameter of the second space 86 is set in such a manner, the peripheral wall 88 pressed by the sealing device 200 easily moves inward in a radial direction when sealing the leading end portion 77, thereby sealing a part of the peripheral wall 88 with another part thereof. In regard to a material included in the leading end portion 77 (the wing member 20), any material may be employable as long as it can be easily sealed by the sealing device 200. Those numerated as the material of the wing member 20 according to the first embodiment may be appropriately applied.

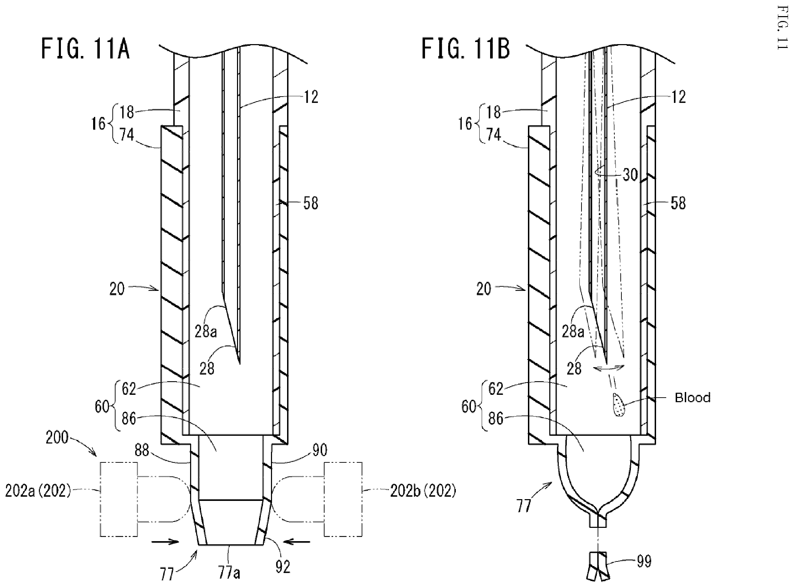

Furthermore, the leading end portion 77 protrudes relatively long toward the leading end (see also Fig. 11A) so that a hold-and-weld mechanism 202 (see Fig. 8) of the sealing device 200 can approach from the outside. For example, it is preferable that a length of the leading end portion 77 protruding from the shaft 74 (axial length) is longer than an outer diameter of the leading end portion 77. Accordingly, the hold-and-weld mechanism 202 can sandwich
the leading end portion 77 to seal (cut and weld) the same sufficiently without interfering with the shaft 74. More specifically, it is preferable that the protruding length of the leading end portion 77 is in a range of 3 to 20 mm. Setting the protruding length of the leading end portion 77 shorter than 3 mm may cause unstable mounting of the cap 24 and insufficient sealing performance of the sealing device 200. Conversely, setting the protruding length of the leading end portion 77 longer than 20 mm shortens an exposed part of the needle body 12, which makes it difficult to insert the needle body 12.

According to this embodiment, in sealing the leading end portion 77, the peripheral wall 88 is pressurized and heated from the outside of the leading end portion 77 so as to be cut and welded. Therefore, an inner face (or an inner layer) of the peripheral wall 88 may be provided with a welding promotion means so that the inner face can be easily welded to the peripheral wall 88 disposed in its periphery. Examples of the welding promotion means include providing a thermal adhesion layer coated with a material which is to be thermally welded at a low temperature, or forming the peripheral wall 88 thick so as to promote a flow of a base material by heating.

Returning to Fig. 8, it is possible to use a device that simultaneously cuts and welds the tube 26 as the sealing device 200 that closes the protector 16. A known device may be applied as the sealing device 200.

The sealing device 200 includes, for example, the hold-and-weld mechanism 202; a gripping portion 204 which is to be gripped by the healthcare professional; an operation grip 206 provided in a front side of the gripping portion 204; and a drive transmission unit 208 configured to open and close the hold-and-weld mechanism 202 based on operation of the operation grip 206. An inner portion of the sealing device 200 is provided with a high-frequency generating device (not illustrated) which applies a high frequency to a pair of stays 202a, 202b of the hold-and-weld mechanism 202. The stay 202a serves as a fixing portion that arranges and fixes the tube 26 and the leading end portion 77 before holding, while the stay 202b serves as a movable portion that comes close to the stay 202a based on a drive force from the drive transmission unit 208 so as to hold the tube 26 and the leading end portion 77 between the stay 202a. Applying a high frequency to the stays 202a, 202b when they hold the tube 26 and the leading end portion 77 between each other causes the stays 202a, 202b to carry out induction hardening.

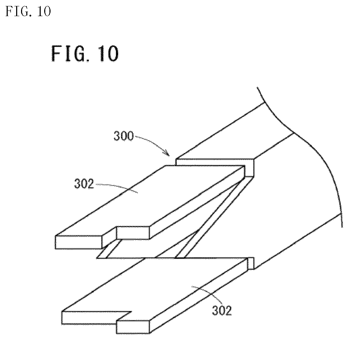

As a matter of course, a device that seals the leading end portion 77 is not limited to the sealing device 200 that generates a high frequency. For example, as illustrated in Fig. 10, a clamp 300 having a pair of grippers 302 can be applied as the device that seals the leading end portion 77. The clamp 300 has a function of closing the leading end portion 77, imparting blood-liquid tightness . Other examples of the device that seals include a heating device, an ultrasonic welding device, a laser device, an adhesive injection device, a stapler, a clipping device, a nipper, a pliers (including roller pliers), and a scissors.

The medical needle 10A according to the second embodiment is basically configured as described above, and its function effect will now be described.

After completion of blood donation (after use), the medical needle 10A is operated by a healthcare professional to retract the hub 14 relative to the protector 16 so as to guide the needle body 12 to a housing space 60 of the protector 16 (see Fig. 9A) . After housing the needle body 12 in the housing space 60, as illustrated in Figs. 9B and 11A, the healthcare professional seals the leading end portion 77 of the protector 16 using the sealing device 200. Inother words, the healthcare professional arranges the leading end portion 77 between the stays 202a, 202b of the hold-and-weld mechanism 202 and operates the operation grip 206 so as to bring the stays 202a, 202b close to each other and to weld the inner face of the peripheral wall 88.

A position to be welded of the leading end portion 77 in an axial direction may be determined by the healthcare professional at his/her discretion, but a boundary of the connecting portion-in-base end 90 and tapered portion-in-leading end 92 is preferably used as a mark. Since the tapered portion-in-leading end 92 narrows an inner diameter of the second space 86, a part of the peripheral wall 88 can be tightly adhered to another part thereof more easily.

Accordingly, as illustrated in Fig. 11B, a cut piece 99 in a leading end of the leading end portion 77 is cut off, and a part of the peripheral wall 88 closer to a base end than the cut position is adhered to another part thereof in an airtight manner so as to close the second space 86 (a leading end of the housing space 60) . Therefore, in a case where blood is attached to an outer periphery of the needle body 12 (or in a case where blood is held by a flow passage 30), even when the blood is separated from the needle body 12 and spatters into the housing space 60, it is possible to prevent spattering of the blood due to sealing at the leading end of the housing space 60.

At the time of sealing, note that a vicinity of a leading end opening 77a of the leading end portion 77 may be sealed so as not to cause the cut piece 99. Alternatively, at the time of sealing, the leading end portion 77 may be welded instead of being cut off. In this case, it is preferable that the hold-and-weld mechanism 202 holds the leading end portion 77 with a light holding force, and the hold-and-weld mechanism 202 is caused to move relatively to the leading end portion 77 along the axial direction of the leading end portion 77 so as to broaden a to-be-welded range in the peripheral wall 88.

As described above, the medical needle 10A according to the second embodiment includes the leading end portion 77, the to-be-sealed portion (blood receiving portion). Accordingly, it is possible to seal the leading end portion 77 after the needle body 12 is housed in the housing space 60 and to close the leading end of the housing space 60. Therefore, it is possible to prevent spattering of the blood held by the needle body 12. Thus, it is possible to enhance hygiene of the medical needle 10A at the time of disposal, which eliminates inconveniences such that medical institutions and other medical devices are contaminated with blood.

In this case, the diameter of the second space 86 of the leading end portion 77 is smaller than the diameter of the first space 62 at a position where the needle tip 28 is housed. Accordingly, in the medical needle 10A, a part of the peripheral wall 88 in the leading end portion 77 is easily brought into contact with another part thereof at the time of sealing, which leads to a preferable sealing. Furthermore, since the protector 16 includes the housing cylinder 18 and the wing member 20, exposure of the needle tip 28 can be prevented by the rigid housing cylinder 18, and the leading end portion 77 of the wing member 20 can be easily sealed, which causes a closed state of the housing space 60.

The medical needle 10A according to the second embodiment is not limited to the above embodiment, and various modifications and applications may be employed. For example, the protector 16 is not limited to the structure including the housing cylinder 18 and the wing member 20, and may be molded in an integrated manner.

Furthermore, the to-be-sealed portion of the protector 16 which is to be sealed is not limited to the shape of the leading end portion 77, and may take various shapes. For example, the to-be-sealed portion maybe formed in a square cylindrical shape, or a shape in which a part of the peripheral wall 88 in a circumferential direction is cut out, or may be formed in a hemispherical shape (in other words, the to-be-sealed portion may not be a cylinder). The to-be-sealed portion may be made not only of the elastic materials numerated as the materials of the wing members 20, 20A but also of various materials that can be sealed easily by an appropriate sealing unit. Still further, the to-be-sealed portion may not be provided to the leading end of the protector 16 as long as the to-be-sealed portion is positioned in a side closer to the leading end than the needle tip 28 of the needle body 12.

## Claims

1. A medical needle (10, 10A), comprising:
a needle body (12); and
a protector (16, 16A, 16B) configured to expose the needle body (12) from a leading end before puncture of the needle body (12), including a housing space (60) which houses the needle body (12) after puncture by relative movement of the needle body (12) toward a base end,
wherein the housing space (60) of the protector (16, 16A, 16B) includes a wall portion (88) configured to form a blood receiving portion (96, 100, 104a, 112) that accumulates blood separated from the needle body (12) by a structure of an inner face of the wall portion (88) or by closing a leading end of the housing space (60) with the needle body (12) being housed in the housing space (60) by sealing,
**characterized in that**
the housing space (60) of the protector (16, 16A, 16B) further includes a leading end portion (76, 77), a diameter of a space of the leading end portion (76, 77) is smaller than the diameter of the housing space at the position where a needle tip (28) is housed, and a leading end opening (76a, 77a) is formed to have a diameter so that the needle body (12) can pass therethrough without touching the leading end opening (76a, 77a) when the needle body (12) retracts.

2. The medical needle (10) according to claim 1, wherein
the blood receiving portion is a blood reservoir (96, 100, 104a, 112) provided to an entire circumference in a circumferential direction of the inner face.

3. The medical needle (10) according to claim 2, wherein
the blood reservoir (96, 100, 104a, 112) is provided to a side closer to the leading end than a needle tip (28) of the needle body (12) housed in the housing space (60).

4. The medical needle (10) according to claim 3, wherein
the blood reservoir (96, 100, 104a, 112) is provided at a position in the leading end of the protector (16, 16A, 16B).

5. The medical needle (10) according to claim 4, wherein
the protector (16, 16A, 16B) includes
a housing cylinder (18, 18A) having rigidity, surrounding a leading end of the needle body (12) while the needle body (12) is housed in the protector (16, 16A, 16B), and
an elastic member (20, 20A) mounted on the housing cylinder (18, 18A), protruding toward the leading end from the housing cylinder (18, 18A).

6. The medical needle (10) according to claim 2, wherein
the blood reservoir (96, 100, 104a) is formed with a barbed portion (94, 102, 104) having a ring-like shape in which a peripheral wall of the protector (16, 16A) protrudes radially inward and toward the base end.

7. The medical needle (10) according to claim 2, wherein
the blood reservoir (96, 100, 104a, 112) is provided with an absorbing portion (98) configured to absorb the blood.

8. The medical needle (10A) according to claim 1, wherein
the blood receiving portion is a to-be-sealed portion capable of closing the leading end of the housing space (60) by sealing.

9. The medical needle (10A) according to claim 8, wherein
the to-be-sealed portion includes an elastic portion which is elastically deformable in accordance with pressing force of the sealing.

10. The medical needle (10A) according to claim 8, wherein
the to-be-sealed portion is formed in a cylindrical shape, being provided with a space (86) included as a part of the housing space (60) in an inner portion of the to-be-sealed portion, and
the space (86) has a diameter smaller than a diameter of the housing space (60) at a position where the needle tip (28) is housed.

11. The medical needle (10A) according to claim 8, wherein
the protector (16) includes
a housing cylinder (18) having rigidity, surrounding a leading end of the needle body (12) while the needle body (12) is housed in the protector (16), and
an elastic member (20) mounted on the housing cylinder (18), including the to-be-sealed portion protruding toward the leading end from the housing cylinder (18).

12. A method for closing a protector (16) of a medical needle (10A) according to any of the claims 1-11, the method comprising:
sealing a to-be-sealed portion including a wall portion (88) of the protector (16) provided to a side closer to the leading end than a needle tip (28) of the needle body (12) with the needle body (12) being housed inside the housing space (60) so as to close a leading end of the housing space (60).

## Patentansprüche

1. Medizinische Nadel (10,10A), umfassend:
einen Nadelkörper (12); und
eine Schutzeinrichtung (16, 16A, 16B), die so konfiguriert ist, dass sie den Nadelkörper (12) von einem vorderen Ende aus vor der Punktion des Nadelkörpers (12) freilegt, umfassend einen Gehäuseraum (60), welcher den Nadelkörper (12) nach der Punktion durch eine relative Bewegung des Nadelkörpers (12) zu einem Basisende hin aufnimmt,
wobei der Gehäuseraum (60) der Schutzeinrichtung (16, 16A, 16B) einen Wandabschnitt (88) aufweist, der so konfiguriert ist, dass er einen Blut aufnehmenden Abschnitt (96, 100, 104a, 112) bildet, der Blut ansammelt, der von dem Nadelkörper (12) durch eine Struktur einer Innenfläche des Wandabschnitts (88) oder durch Verschließen eines vorderen Endes des Gehäuseraums (60) getrennt ist, wobei der Nadelkörper (12) durch Abdichtung in dem Gehäuseraum (60) untergebracht ist, **dadurch gekennzeichnet, dass**
der Gehäuseraum (60) der Schutzeinrichtung (16, 16A, 16B) ferner einen vorderen Endabschnitt (76, 77) aufweist, wobei ein Durchmesser eines Raums des vorderen Endabschnitts (76, 77) kleiner ist als der Durchmesser des Gehäuseraums an der Position, an der eine Nadelspitze (28) untergebracht ist, und eine vordere Endöffnung (76a, 77a) so ausgebildet ist, dass sie einen Durchmesser aufweist, sodass der Nadelkörper (12) durch sie hindurchgehen kann, ohne die vordere Endöffnung (76a, 77a) zu berühren, wenn der Nadelkörper (12) zurückgezogen wird.

2. Medizinische Nadel (10) nach Anspruch 1, wobei der Blut aufnehmende Abschnitt ein Blutreservoir (96, 100, 104a, 112) ist, das an einem gesamten Umfang in einer Umfangsrichtung der Innenfläche vorgesehen ist.

3. Medizinische Nadel (10) nach Anspruch 2, wobei das Blutreservoir (96, 100, 104a, 112) an einer Seite vorgesehen ist, die näher an dem vorderen Ende liegt als eine Nadelspitze (28) des Nadelkörpers (12), der in dem Gehäuseraum (60) untergebracht ist.

4. Medizinische Nadel (10) nach Anspruch 3, wobei das Blutreservoir (96, 100, 104a, 112) an einer Position in dem vorderen Ende der Schutzeinrichtung (16, 16A, 16B) vorgesehen ist.

5. Medizinische Nadel (10) nach Anspruch 4, wobei die Schutzeinrichtung (16, 16A, 16B) umfasst einen Gehäusezylinder (18, 18A), der eine Steifigkeit aufweist, der ein vorderes Ende des Nadelkörpers (12) umgibt, während der Nadelkörper (12) in der Schutzeinrichtung (16, 16A, 16B) untergebracht ist, und ein elastisches Element (20, 20A), das an dem Gehäusezylinder (18, 18A) angebracht ist, wobei es in Richtung des vorderen Endes von dem Gehäusezylinder (18, 18A) vorsteht.

6. Medizinische Nadel (10) nach Anspruch 2, wobei das Blutreservoir (96, 100, 104a) mit einem mit Widerhaken versehenen Abschnitt (94, 102, 104) ausgebildet ist, der eine ringartige Form aufweist, bei der eine Umfangswand der Schutzeinrichtung (16, 16A) radial nach innen und in Richtung zu dem Basisende hin vorsteht.

7. Medizinische Nadel (10) nach Anspruch 2, wobei das Blutreservoir (96, 100, 104a, 112) mit einem absorbierenden Abschnitt (98) versehen ist, der zum Absorbieren des Blutes konfiguriert ist.

8. Medizinische Nadel (10A) nach Anspruch 1, wobei der das Blut aufnehmende Abschnitt ein abzudichtender Abschnitt ist, der in der Lage ist, das vordere Ende des Gehäuseraums (60) durch Abdichtung zu verschließen.

9. Medizinische Nadel (10A) nach Anspruch 8, wobei der abzudichtende Abschnitt einen elastischen Abschnitt aufweist, der in Übereinstimmung mit der Druckkraft der Abdichtung elastisch verformbar ist.

10. Medizinische Nadel (10A) nach Anspruch 8, wobei
der abzudichtende Abschnitt in einer zylindrischen Form ausgebildet ist, die mit einem Raum (86) versehen ist, der als ein Teil des Gehäuseraums (60) in einem inneren Abschnitt des abzudichtenden Abschnitts enthalten ist, und
der Raum (86) einen Durchmesser aufweist, der kleiner ist als ein Durchmesser des Gehäuseraums (60) an einer Position, an der die Nadelspitze (28) untergebracht ist.

11. Medizinische Nadel (10A) nach Anspruch 8, wobei
die Schutzeinrichtung (16) umfasst einen Gehäusezylinder (18), der eine Steifigkeit aufweist, der ein vorderes Ende des Nadelkörpers (12) umgibt, während der Nadelkörper (12) in der Schutzeinrichtung (16) untergebracht ist, und
ein elastisches Element (20), das an dem Gehäusezylinder (18) angebracht ist, das den abzudichtenden Abschnitt umfasst, wobei es in Richtung des vorderen Endes von dem Gehäusezylinder (18) vorsteht.

12. Verfahren zum Verschließen einer Schutzeinrichtung (16) einer medizinischen Nadel (10A) nach einem der Ansprüche 1 bis 11, wobei das Verfahren umfasst:
Abdichten eines abzudichtenden Abschnitts, der einen Wandabschnitt (88) der Schutzeinrichtung (16) umfasst, der auf einer Seite vorgesehen ist, die näher an dem vorderen Ende liegt als eine Nadelspitze (28) des Nadelkörpers (12), wobei der Nadelkörper (12) innerhalb des Gehäuseraums (60) untergebracht ist, um ein vorderes Ende des Gehäuseraums (60) zu verschließen.

## Revendications

1. Aiguille à usage médical (10, 10A), comprenant :
un corps d'aiguille (12) ; et
une protection (16, 16A, 16B) configurée pour exposer le corps d'aiguille (12) à partir d'une extrémité d'attaque avant une ponction du corps d'aiguille (12), comportant un espace d'admission (60) qui admet le corps d'aiguille (12) après une ponction par un mouvement relatif du corps d'aiguille (12) en direction d'une extrémité de base,
dans laquelle l'espace d'admission (60) de la protection (16, 16A, 16B) comporte une partie de paroi (88) configurée pour former une partie de réception de sang (96, 100, 104a, 112) qui accumule du sang séparé du corps d'aiguille (12) par une structure d'une face interne de la partie de paroi (88) ou en fermant une extrémité d'attaque de l'espace d'admission (60) avec le corps d'aiguille (12) qui est admis dans l'espace d'admission (60) par scellage, **caractérisée en ce que**
l'espace d'admission (60) de la protection (16, 16A, 16B) comporte en outre une partie d'extrémité d'attaque (76, 77), un diamètre d'un espace de la partie d'extrémité d'attaque (76, 77) est plus petit que le diamètre de l'espace d'admission au niveau de la position où une pointe d'aiguille (28) est admise, et une ouverture d'extrémité d'attaque (76a, 77a) est formée pour présenter un diamètre de sorte que le corps d'aiguille (12) puisse passer au travers sans toucher l'ouverture d'extrémité d'attaque (76a, 77a) lorsque le corps d'aiguille (12) se rétracte.

2. Aiguille à usage médical (10) selon la revendication 1, dans laquelle
la partie de réception de sang est un réservoir de sang (96, 100, 104a, 112) disposé sur une circonférence totale dans une direction circonférentielle de la face interne.

3. Aiguille à usage médical (10) selon la revendication 2, dans laquelle
le réservoir de sang (96, 100, 104a, 112) est disposé sur un côté plus proche de l'extrémité d'attaque qu'une pointe d'aiguille (28) du corps d'aiguille (12) admise dans l'espace d'admission (60).

4. Aiguille à usage médical (10) selon la revendication 3, dans laquelle
le réservoir de sang (96, 100, 104a, 112) est disposé au niveau d'une position dans l'extrémité d'attaque de la protection (16, 16A, 16B).

5. Aiguille à usage médical (10) selon la revendication 4, dans laquelle
la protection (16, 16A, 16B) comporte
un cylindre d'admission (18, 18A) présentant une rigidité, entourant une extrémité d'attaque du corps d'aiguille (12) alors que le corps d'aiguille (12) est admis dans la protection (16, 16A, 16B), et
un élément élastique (20, 20A) monté sur le cylindre d'admission (18, 18A), faisant saillie en direction de l'extrémité d'attaque à partir du cylindre d'admission (18, 18A) .

6. Aiguille à usage médical (10) selon la revendication 2, dans laquelle
le réservoir de sang (96, 100, 104a) est formé avec une partie barbelée (94, 102, 104) présentant une forme annulaire dans laquelle une paroi périphérique de la protection (16, 16A) fait saillie radialement vers l'intérieur et vers l'extérieur de l'extrémité de base.

7. Aiguille à usage médical (10) selon la revendication 2, dans laquelle
le réservoir de sang (96, 100, 104a, 112) est doté d'une partie absorbante (98) configurée pour absorber le sang.

8. Aiguille à usage médical (10A) selon la revendication 1, dans laquelle
la partie de réception de sang est une partie devant être scellée apte à fermer l'extrémité d'attaque de l'espace d'admission (60) par scellage.

9. Aiguille à usage médical (10A) selon la revendication 8, dans laquelle
la partie devant être scellée comporte une partie élastique qui est élastiquement déformable conformément à une force de pression du scellage.

10. Aiguille à usage médical (10A) selon la revendication 8, dans laquelle
la partie devant être scellée présente une forme cylindrique, qui est dotée d'un espace (86) inclus en tant que partie de l'espace d'admission (60) dans une partie interne de la partie devant être scellée, et
l'espace (86) présente un diamètre plus petit qu'un diamètre de l'espace d'admission (60) au niveau d'une position où la pointe d'aiguille (28) est admise.

11. Aiguille à usage médical (10A) selon la revendication 8, dans laquelle
la protection (16) comporte
un cylindre d'admission (18) présentant une rigidité, entourant une extrémité d'attaque du corps d'aiguille (12) alors que le corps d'aiguille (12) est admis dans la protection (16), et
un élément élastique (20) monté sur le cylindre d'admission (18), comportant la partie devant être scellée faisant saillie en direction de l'extrémité d'attaque à partir du cylindre d'admission (18).

12. Procédé de fermeture d'une protection (16) d'une aiguille à usage médical (10A) selon les revendications 1-11, le procédé comprenant :
le scellage d'une partie devant être scellée comportant une partie de paroi (88) de la protection (16) disposée sur un côté plus proche de l'extrémité d'attaque qu'une pointe d'aiguille (28) du corps d'aiguille (12) avec le corps d'aiguille (12) qui est admis à l'intérieur de l'espace d'admission (60) de manière à fermer une extrémité d'attaque de l'espace d'admission (60).
